# DEMANDE DE BREVET EUROPEEN

(11) **EP 3 111 739 A1**
(43) Date de publication de la demande: **04.01.2017**
(21) Numéro de dépôt: 16177624.0
(22) Date de dépôt: 01.07.2016
(51) Int. Cl.: A01C 3/02, C12M 1/107

(54) **DISPOSITIF POUR LA METHANISATION PAR VOIE SECHE DE MATIERE ORGANIQUE COMPRENANT DU FUMIER**

(30) Priorité: 01.07.2015 FR 1556227
(71) Demandeur: EASYMETHA, 80440 Boves (FR)
(72) Inventeur: PEULTIER, Philippe, 80080 Amiens (FR)
(74) Mandataire: Balesta, Pierre

(57) **Abrégé**

La présente invention a pour objet un dispositif (1) pour la méthanisation par voie sèche de matière organique (2) comprenant du fumier. Le dispositif comprend au moins un digesteur (3) comprenant au moins une zone de collecte et d'alimentation (4) de la matière organique (2) à digérer, un couloir de digestion (5) ayant une zone de génération de gaz (11) et une zone de collecte du gaz (12) disposée au-dessus de la zone de génération du gaz (11). Le digesteur comprend en outre une zone d'évacuation et de stockage tampon (7) de la matière organique digérée (9) en liaison avec le couloir de digestion.

## Description

La présente invention concerne le domaine technique des dispositifs de méthanisation par voie sèche de matières organiques, ainsi que les procédés de méthanisation par voie sèche mettant en oeuvre lesdits dispositifs.

### Arrière-plan de l'invention

La méthanisation est une transformation de la matière organique qui permet la production de biogaz et de digestat. La valorisation du biogaz permet d'obtenir de l'électricité, de la chaleur, du biométhane, utilisé par exemple comme carburant. La méthanisation permet également la production de digestats valorisables pouvant par exemple être utilisés comme fertilisants et ainsi être épandus.

La méthanisation est un processus naturel de recyclage de la matière organique fermentescible, dans un milieu sans oxygène (anaérobie), due à l'action de nombreux microorganismes qui transforment la matière organique en biogaz et en digestat. La méthanisation comprend quatre grandes étapes : l'hydrolyse, l'acidogénèse, l'acétogénèse et la méthanogénèse. La méthanisation conduit à la production d'un gaz, appelé biogaz, comprenant entre 40% et 70% de méthane (CH₄) et, entre 30% et 50% de dioxyde de carbone, qui est valorisable sous forme d'énergie en électricité grâce à un moteur de cogénération, en chaleur, en biocarburant ou qui peut être injecté dans le réseau de gaz naturel après une étape de purification devenant alors du biométhane.

La décomposition de la matière organique fournit également un produit riche en humus et partiellement stabilisé, appelé digestat, qu'il est ensuite possible de valoriser comme fertilisant naturel et amendement organique pour l'agriculture.

La méthanisation présente ainsi de nombreux avantages : la production locale et la vente d'électricité, de chaleur et de biométhane, la production d'un engrais renouvelable pour l'exploitation, la réalisation d'économies d'engrais grâce à une meilleure disponibilité de l'azote et du phosphore.

Différentes techniques de digestion existent : la digestion par voie liquide (également désignée sous le terme d'infiniment mélangé) et la digestion par voie sèche, notamment continue ou discontinue.

La digestion par voie liquide se fait généralement dans un digesteur comprenant une cuve de digestion en acier ou en béton, enterrée ou non, close hermétiquement et dont la température est maintenue soit à 37°C-39°C (régime mésophile), soit à 45-55°C (régime thermophile). La quantité de matière sèche dans la matière organique à traiter, laquelle matière organique comprend également des fluides, est généralement inférieure à 12% en poids. Le mélange à l'intérieur de la cuve est maintenu homogène grâce à un brassage continu qui a aussi pour fonction d'éviter la formation de la croûte en surface et la sédimentation des matières, et facilite également le dégazage.

La digestion par voie sèche continue ou discontinue se déroule en régime mésophile ou thermophile avec un temps de séjour au minimum de l'ordre de 20 jours.

Les matières organiques à digérer peuvent comprendre des végétaux, notamment des déchets végétaux (tontes, herbes, feuillages), bio-déchets alimentaires (biscuits, légumes,...), des lisiers ou des fumiers (paille éventuellement mélangée avec des lisiers et/ou déjections animales), par exemple des lisiers ou des fumiers de porcs, des lisiers ou des fumiers de bovins, des sous-produits agricoles, par exemple des résidus de céréales, de la menue paille, de la paille, des huiles végétales. Il a été constaté que les matières organiques à digérer dites « sèches », c'est-à-dire comprenant au moins 10% en masse par rapport à leur masse totale de matières solides procurent de meilleurs rendements énergétiques. En effet, la voie liquide décrite ci-dessus engendre des coûts de fonctionnement élevés en raison des machines de pompages et des agitateurs nécessaires à son bon fonctionnement. Ce type de digestion est adapté aux très grosses productions agricoles ou collectives mais convient moins à celles de petites ou moyennes tailles.

Le digestat est stockable et épandable avec un épandeur à fumier (une table d'épandage est préconisée). De plus, les coûts d'investissements sont globalement moins élevés que par la voie humide.

FR 2 481 873 et FR 2 495 887 ont pour objet des digesteurs dans lesquels la progression de la matière organique est assurée par une vis sans fin ou par des râteaux.

FR 2 475 524 a pour objet un digesteur dans lequel la progression de la matière organique est assurée par des pompes aptes également à l'homogénéiser. La matière sèche de la matière organique n'est pas précisée mais doit être faible, et notamment inférieure ou égale à 10%, car elle peut être pompée de la zone de stockage pour alimenter la zone de digestion. Dans ce but, la matière organique est broyée et mélangée avec de l'eau.

Les digesteurs agencés pour effectuer une méthanisation par voie sèche ont pour inconvénients que les dispositifs d'acheminement de la matière organique à digérer tout au long de la zone de digestion sont soumis à un milieu très agressif (acide et ce sous une chaleur de l'ordre de 37°C au moins) de sorte que ces derniers s'usent vite et nécessitent un entretien régulier, voire leurs renouvellements, ce qui peut s'avérer coûteux. De plus, ces digesteurs sont complexes et donc coûteux à fabriquer.

Il existe ainsi un besoin pour des digesteurs de méthanisation par voie sèche simples à mettre en oeuvre, dont la maintenance est peu coûteuse et simplifiée, et capable de s'adapter à la taille des exploitations agricoles.

### Objet et résumé de l'invention

La présente invention pallie tout ou partie des problèmes précités en ce qu'elle a pour objet selon un premier aspect un dispositif pour la méthanisation par voie sèche de matière organique, comprenant au moins un digesteur, lequel digesteur comprend avantageusement :
- au moins une zone de collecte et d'alimentation de la matière organique à digérer comprenant du fumier notamment apte à tenir en tas ;
- un couloir de digestion comprenant :
   - une zone de génération de gaz destinée à recevoir la matière organique ayant été mouillée avec un fluide, notamment ayant une température supérieure ou égale à 35°C ;
   - une zone de collecte du gaz disposée au-dessus de la zone de génération du gaz pour collecter le biogaz produit dans la zone de génération de gaz ;
   - une zone d'évacuation et de stockage tampon de la matière organique digérée (digestat).

Ledit couloir de digestion comprend au moins un orifice d'admission de la matière organique à digérer pour son admission de la zone de collecte et d'alimentation dans la zone de génération de gaz, ledit orifice d'admission comprend éventuellement un dispositif de réglage de la taille de son ouverture amovible.

Ledit couloir de digestion comprend également au moins un orifice d'évacuation de la matière organique digérée pour son évacuation de la zone de génération de gaz dans la zone d'évacuation et de stockage tampon, ledit orifice d'évacuation comprend un dispositif de fermeture et d'ouverture amovible.

Avantageusement, il a été observé de manière surprenante que la matière organique brute comprenant du fumier, notamment disposée en forme de tas, arrosée avec un fluide (eau, déjections animales liquides, autres déchets organiques fluides, ou leur mélange), et ce tout en observant un taux de matière sèche élevé, adopte au fur et à mesure de sa maturation, et donc de sa digestion, un comportement rhéologique particulier lui permettant de se déplacer sous l'effet de son propre poids (déplacement gravitaire). On observe que la matière organique mouillée comprenant du fumier se comporte tel un solide en son centre et tel un liquide sur sa périphérie et donc en contact avec les parois du couloir de digestion ce qui permet son auto-déplacement.

Ce comportement de la matière organique n'est pas observable si cette dernière ne comprend pas de fumier et est par exemple constituée uniquement de lisiers et/ou de déjections animales et/ou de déchets verts.

Ledit au moins un digesteur selon l'invention utilise ainsi ce comportement de la matière organique mouillée avec un fluide comprenant du fumier afin d'obtenir le déplacement de cette dernière dans le digesteur entre son orifice d'admission et ledit au moins un orifice d'évacuation. Les dispositifs mécaniques d'avancement de la matière organique utilisés dans l'état de la technique, telles les vis sans fin ou les râteaux, nécessitant de l'énergie pour leur fonctionnement et coûteux pour leur maintenance, ne sont pas utiles dans le dispositif selon l'invention. Le déplacement de la matière organique mouillée avec un effluent dans le digesteur est obtenu sans l'aide de moyens mécaniques permettant son avancement.

Avantageusement, le digesteur selon l'invention peut traiter une matière organique brute qui n'a pas été au préalable broyée ou déchiquetée, en particulier qui n'a pas été mélangée avec de l'eau afin de la rendre pompable.

Avantageusement, le digesteur selon l'invention comprend également une zone d'évacuation et de stockage tampon en sortie du couloir de digestion afin d'évacuer régulièrement dudit couloir de digestion la matière organique digérée ou digestat. Cette zone tampon permet non seulement de maîtriser/réguler la sortie de la matière organique digérée mais également de réguler la durée de méthanisation dans le couloir de digestion puisque ce dernier ne comprend aucun dispositif de déplacement de la matière organique.

On entend au sens de la présente invention par « fumier », de la paille et/ou de la menue paille, éventuellement mélangée(s) avec du lisier et/ou des déjections animales.

De préférence, la matière organique selon l'invention comprend du fumier éventuellement en mélange avec du lisier et/ou des déjections animales, en particulier issu(e)(s) des élevages bovins, porcins, ovins, volailles ou leur mélange ; et/ou des matières végétales, telles que des résidus de récolte ou autres déchets verts (tontes, feuillages, herbes,...), des déchets de céréales, de fruits et de légumes ; et/ou des déchets de l'industrie agro-alimentaire (biscuits,...) et/ou encore des collectivités (cantines,...). Encore de préférence, la matière organique selon l'invention est brute, notamment elle n'est pas broyée.

De préférence, la matière organique selon l'invention comprend au moins 30% de fumier en masse par rapport à sa masse totale, notamment au moins 50% de fumier en masse par rapport à sa masse totale.

La matière organique mouillée avec un fluide selon l'invention n'est pas apte à être pompée car elle est trop riche en fibres, et présente un taux de matière sèche important

Le taux de matière sèche est calculé en rapportant la masse totale de la matière organique mouillée par un fluide (par exemple prélevée dans le couloir de digestion) sur la masse totale de matière sèche. La masse totale de la matière sèche peut être obtenue après avoir séché pendant plusieurs heures, notamment 6h à 100°C en étuve, la matière organique mouillée.

De préférence, ledit fluide est de l'eau ou comprend des lisiers et/ou des déjections animales, notamment issus des élevages bovins, porcins, volailles ou ovins, ou encore leur mélange, et/ou des fluides issus de l'industrie agro-alimentaire, ces derniers pouvant être mélangés avec de l'eau.

De préférence, la matière organique est mouillée, notamment arrosée, par ledit fluide ayant une température supérieure ou égale à 30°C, encore de préférence supérieure ou égale à 35°C.

Le mode de digestion selon l'invention peut être mésophile ou thermophile.

De préférence, la matière organique comprenant du fumier selon l'invention tient en tas lorsqu'elle est disposée dans la zone de collecte et d'alimentation préalablement à son arrosage par un fluide. De préférence, la matière organique est disposée sous forme de dôme dans ladite zone de collecte et d'alimentation puis arrosée jusqu'à ce que ledit tas, en particulier le dôme, s'abaisse.

De préférence, le taux de matière sèche de la matière organique, mouillée par un effluent, et apte à se déplacer de façon gravitaire est supérieur ou égal à 12%, encore de préférence supérieur ou égal à 15%, encore plus préférentiellement supérieur ou égal à 18%.

Selon un mode de réalisation, le dispositif de fermeture et d'ouverture, comprend un volet apte à pivoter autour d'un axe (F) sensiblement parallèle à l'axe de déplacement (D) de la matière organique dans le couloir de digestion. De préférence, l'axe de déplacement (D) de la matière organique dans le couloir de digestion est sensiblement parallèle à l'axe longitudinal (L) dudit couloir de digestion.

Selon un autre mode de réalisation, le dispositif de fermeture et d'ouverture amovible comprend un organe de fermeture, par exemple une lame guillotine, apte à fermer ledit au moins un orifice d'évacuation, éventuellement lesdits deux ou trois orifices d'évacuation alignés verticalement (décrits ci-après), en se déplaçant selon une direction transversale, notamment perpendiculaire, à l'axe de déplacement (D) de la matière organique mouillée, en particulier perpendiculairement à l'axe longitudinal (L) du couloir de digestion.

Ledit dispositif peut être une vanne guillotine à fermeture rapide.

Selon un premier mode de réalisation, la zone d'évacuation et de stockage tampon comprend également un orifice de sortie de la matière organique digérée (digestat), éventuellement pourvue d'un dispositif de fermeture et d'ouverture amovible, notamment identique au dispositif de fermeture et d'ouverture amovible dudit au moins un orifice d'évacuation du couloir de digestion.

Selon un second mode de réalisation, éventuellement combiné avec le premier mode de réalisation, la zone d'évacuation et de stockage tampon est une fosse pourvue éventuellement d'un élément de toit amovible.

La matière organique mouillée par ledit fluide est digérée pendant au moins 20 jours dans le couloir de digestion.

De préférence, le biogaz produit comprend environ de 40% à 70% en volume de méthane, de 30% à 50% en volume de dioxyde de carbone, en particulier des traces d'hydrogène sulfuré et d'eau.

Selon un mode de réalisation, le biogaz produit est épuré pour produire un biogaz comprenant au moins 97% en volume de méthane.

Le biogaz produit comprenant du méthane à plus de 90% en volume est ensuite compressé, puis odorisé afin de le rendre perceptible olfactivement. Ce biogaz ainsi traité peut ainsi fournir les réseaux d'alimentation classiques en gaz naturel (type GrDF).

Le digestat produit conserve les éléments fertilisants, et donc un potentiel humifère important.

Ledit dispositif de méthanisation comprend des moyens de collecte et de stockage du biogaz produit.

Le couloir de digestion, et éventuellement la zone d'évacuation et de stockage tampon, comprend/comprennent des moyens de collecte et d'acheminement du biogaz vers une zone de stockage du biogaz.

Dans une variante, le couloir de digestion comprend au moins une cloison de séparation disposée dans ledit couloir de digestion afin de former au moins deux chambres de digestion, notamment en liaison fluidique l'une avec l'autre, ladite cloison de séparation comprend éventuellement des moyens de chauffage pour le chauffage de la matière organique, notamment à une température supérieure ou égale à 35°C.

De préférence, lesdites chambres de digestion sont disposées en parallèle entre lesdits orifices d'admission et d'évacuation du couloir de digestion.

De préférence, chaque chambre de digestion comprend un orifice d'admission correspondant à l'un des orifices d'admission du couloir de digestion.

Avantageusement, lesdites au moins deux chambres de digestion partagent une cloison de séparation commune.

Ladite cloison de séparation permet d'améliorer le chauffage de la matière organique à digérer, et ce de façon homogène. La matière organique est en effet difficile à chauffer de manière homogène car elle comprend des végétaux, tels que la paille, qui sont connus pour être des matériaux isolants thermiquement.

Cette cloison crée également du cisaillement sur la matière organique ce qui favorise son brassage.

Dans un mode de réalisation, ladite au moins une cloison de séparation s'étend dans la zone de collecte et d'alimentation et sépare cette dernière en au moins deux sous-zones.

Dans une variante, le couloir de digestion comprend des parois latérales gauche et droite et une paroi de fond, et ledit dispositif de méthanisation comprend un moyen de raclement d'au moins l'une desdites parois latérales et de fond disposé librement. De préférence, le moyen de raclement comprend au moins une grille disposée librement dans la matière organique et se déplaçant le long du couloir de digestion, de préférence dans au moins une chambre de digestion, sous l'effet du déplacement libre de la matière organique.

Ledit moyen de raclement peut être disposé dans la zone de collecte et de stockage puis entrainé par la matière organique comprenant du fumier et mouillée à travers au moins l'un desdits orifices d'admission dudit couloir de digestion puis dans ce dernier, notamment dans l'une desdites chambres de digestion, voire dans les deux chambres de digestion à la fois lorsque le moyen de raclement comprend deux grilles reliées dans leurs parties supérieures telles que décrites ci-après.

Ce moyen de raclement, en particulier lorsqu'il s'agit d'une grille a pour effet d'homogénéiser l'avancement de la matière organique dans le couloir de digestion et d'éviter que la matière organique ne colle aux parois latérales et de fond du couloir ou encore à la cloison de séparation. Ladite au moins une grille permet également de dégazer la matière organique.

Selon un mode de réalisation, les parois latérales gauche et droite sont parallèles selon tout ou partie de leur longueur et/ou convergent selon une partie de leur longueur vers l'orifice d'évacuation. Cette dernière disposition favorise le cisaillement de la matière organique mouillée et donc son homogénéisation.

De préférence, le moyen de raclement est dimensionné par rapport aux dimensions du couloir de digestion, et notamment par rapport à celles desdites chambres de digestion, en sorte de racler les parois latérales et de fond du couloir de digestion, et notamment au moins l'une des parois de la cloison de séparation.

Dans un premier mode de réalisation, le moyen de raclement comprend deux grilles reliées dans leurs parties supérieures par un bras, lequel bras en fonctionnement se déplace au-dessus de la cloison de séparation, chaque chambre de digestion recevant une grille.

De préférence, le moyen de raclement, en particulier chaque grille selon l'invention, comprend une partie supérieure et une partie inférieure montées sur un ressort de rappel en sorte d'être maintenues l'une contre l'autre au repos et d'être aptes à s'écarter l'une de l'autre sous l'effet d'une contrainte jusqu'à tenir dans un même plan en pivotant autour de l'axe transversal séparant la partie supérieure de la partie inférieure.

Dans un second mode de réalisation, inverse au mode précédent, le moyen de raclement comprend au moins une grille, laquelle grille comprend une partie supérieure et une partie inférieure montées l'une par rapport à l'autre à l'aide d'un dispositif de rappel, tel un ressort de rappel, en sorte qu'en position déployée, et donc au repos, les parties supérieure et inférieure soient sensiblement dans un même plan, et qu'en position pliée, la partie supérieure soit apte à pivoter et se rabattre vers la partie inférieure.

De préférence, ledit moyen de raclement est disposé dans la zone de collecte et d'alimentation et entraîné vers le couloir de digestion, en particulier vers au moins une chambre de digestion par le déplacement gravitaire de la matière organique.

Avantageusement, la grille peut réduire son encombrement, en étant dans une position pliée, lors de son passage, de la zone de collecte et de stockage vers le couloir de digestion, à travers ledit au moins un orifice d'admission du couloir de digestion.

Cette disposition évite d'agencer une ouverture d'introduction d'une grille dans le couloir de digestion, laquelle ouverture est susceptible d'introduire de l'air et ainsi de perturber la digestion en phase anaérobie.

Dans un mode de réalisation, le dispositif de méthanisation comprend une ouverture de récupération dudit au moins un moyen de raclement, disposée près dudit au moins un orifice d'évacuation du couloir de digestion.

Ledit moyen de raclement est ainsi introduit dans le couloir de digestion en disposant ce dernier dans la zone de collecte et de stockage.

De préférence, les ouvertures de la grille ont au moins une dimension supérieure à un centimètre.

De préférence, le dispositif selon l'invention comprend au moins deux moyens de raclement, encore de préférence au moins quatre moyens de raclement.

De préférence, le dispositif de méthanisation comprend au moins un moyen de raclement disposé librement dans une chambre de digestion délimitée au moins en partie par la cloison de séparation.

Dans une variante, le dispositif de méthanisation comprend des moyens de chauffage disposés dans au moins l'une desdites parois latérales et de fond et/ou la cloison de séparation, et comprend un module de cogénération configuré pour être alimenté au moins en partie par le biogaz produit dans la zone de collecte et pour alimenter en chaleur lesdits moyens de chauffage.

De préférence, le module de cogénération comprend un moteur entraînant un alternateur-générateur de courant électrique. De préférence, le moteur de cogénération est un moteur à gaz.

Dans une variante, le dispositif de méthanisation comprend une ouverture d'admission dudit au moins un moyen de raclement, disposée à proximité dudit orifice d'admission du couloir de digestion, et une ouverture de récupération dudit au moins un moyen de raclement, disposée près dudit au moins un orifice d'évacuation du couloir de digestion.

L'entretien du couloir de digestion est ainsi facilité puisque l'opérateur peut récupérer les moyens de raclement pour leur nettoyage via ladite ouverture de récupération et/ou insérer de nouveaux moyens de raclement par ladite ouverture d'admission, sans ouvrir totalement ledit couloir de digestion et donc perturber la digestion, et notamment ôter totalement l'élément de toit du couloir de digestion.

Il est possible d'introduire des moyens de raclement dans le digesteur par ladite ouverture d'admission dans le couloir de digestion mais également en disposant au moins un moyen de raclement dans la zone de collecte et de stockage (notamment disposé à plat dans cette dernière).

Dans une variante, la partie supérieure du couloir de digestion est fermée à l'aide d'un élément de toit de manière amovible, de préférence lesdites ouvertures d'admission et de récupération des moyens de raclement sont disposées dans ledit élément de toit.

Dans une variante, la zone de collecte et d'alimentation, le couloir de digestion, et la zone d'évacuation et de stockage tampon, sont au moins partiellement enterrés, de préférence totalement enterrés.

De préférence, la zone de collecte et d'alimentation, le couloir de digestion, la cloison de séparation, et la zone d'évacuation et de stockage tampon sont au moins en partie en béton, ledit béton est de préférence isolé thermiquement afin de limiter les pertes de chaleur.

L'enterrement au moins partiel desdites zones du dispositif de méthanisation et du couloir de digestion limite également les pertes de chaleur.

Dans une variante, le dispositif de méthanisation comprend selon une portion déterminée de la longueur du couloir de digestion, une zone d'évent laquelle est recouverte par au moins une membrane imperméable au biogaz, de préférence ladite membrane est recouverte d'une membrane de protection.

Ladite zone d'évent permet au gaz de s'échapper en cas d'explosion en créant une zone de « faiblesse » orientée dans le dispositif de méthanisation. La ou les membrane(s) superposée(s) se déchirera/déchireront sous l'effet de l'explosion sans endommager le restant de la structure du dispositif de méthanisation.

Ladite zone d'évent s'étend sur au moins un tiers de la longueur du couloir de digestion.

Dans une variante, le couloir de digestion comprend au moins deux, de préférence au moins trois, orifices d'évacuation, alignés verticalement.

Cette disposition permet avantageusement une sortie de manière homogène de la matière organique digérée.

Dans une variante, la zone d'évacuation et de stockage tampon comprend un orifice de sortie de la matière organique s'étendant du fond de ladite zone d'évacuation et de stockage tampon pourvu d'un dispositif de fermeture et d'ouverture amovible, notamment tel que déjà décrit dans le présent texte.

Cette disposition facilite la sortie et donc l'évacuation de la matière organique digérée en évitant que cette dernière ne sédimente en certains endroits de la zone d'évacuation et de stockage tampon.

Dans une variante, le dispositif de méthanisation comprend au moins deux digesteurs, lesdits deux digesteurs ayant au moins une paroi latérale commune.

Cette disposition favorise les économies d'énergie pour le chauffage de la matière organique. Par ailleurs, l'entretien du dispositif selon l'invention est facilité puisqu'il est possible d'accéder à un couloir de digestion sans stopper le travail d'un autre couloir de digestion parallèle.

Enfin, cette disposition permet de s'adapter à la taille des exploitations agricoles ou équivalentes, en particulier si leurs productions de matières organiques ne sont pas constantes.

Les couloirs de digestion sont de préférence disposés en parallèle.

La présente invention a pour objet selon un second aspect de l'invention un procédé de méthanisation par voie sèche de matière organique comprenant avantageusement les étapes définies ci-après, mises en oeuvre à l'aide du dispositif de méthanisation décrit en référence selon un premier aspect de l'invention :
a) une étape de collecte et d'alimentation de la matière organique à digérer comprenant du fumier, notamment pouvant tenir en tas pour la formation d'un tas de matière organique, tel un dôme, dans une zone de collecte et d"alimentation ; et
b) une étape d'arrosage de la matière organique disposée dans la zone de collecte et d'alimentation à l'aide d'un fluide, notamment ayant une température supérieure ou égale à 30°C, encore de préférence supérieure ou égale à 35°C ; et
c) une étape de transfert de la matière organique de la zone d'alimentation et de collecte vers un couloir de digestion ayant un orifice d'admission de la matière organique et au moins un orifice d'évacuation de la matière organique, l'avancée de la matière organique dans le couloir de digestion entre les orifices d'admission et d'évacuation est obtenue par le déplacement gravitaire de la matière organique, le couloir de digestion comprenant un dispositif de fermeture et d'ouverture amovible dudit au moins un orifice d'évacuation ; et
d) ledit couloir de digestion comprend en outre une zone de génération de gaz destinée à recevoir la matière organique ayant été mouillée avec un fluide et une zone de collecte du gaz disposée au-dessus de la zone de génération du gaz dans ledit couloir de digestion pour collecter le biogaz produit dans la zone de génération de gaz ; et
e) une étape de collecte du biogaz produit ; et
f) une étape de transfert de la matière organique digérée (digestat) du couloir de digestion vers une zone d'évacuation et de stockage tampon.

De préférence, la matière organique disposée dans la zone de collecte et d'alimentation, notamment en forme de dôme, est arrosée à l'aide d'un fluide, en particulier jusqu'à abaissement du tas, notamment du dôme, c'est-à-dire jusqu'à ce que la zone affleurant à la surface du tas et orientée vers l'arrosage soit sensiblement plane, la matière organique mouillée par le fluide contenant trop de matière sèche et de fibres pour pouvoir être pompée.

Dans une variante, le biogaz produit alimente un moteur de cogénération générant de l'électricité et de la chaleur, ladite chaleur étant utilisée comme moyen de chauffage d'au moins l'une desdites parois du couloir de digestion, de préférence des parois latérales et de fond, et/ou comme moyen de chauffage de ladite cloison de séparation du couloir de digestion.

La présente invention a pour objet, selon un troisième aspect, une installation comprenant un dispositif de méthanisation selon l'une quelconque des variantes de réalisation en référence à un premier aspect, ladite installation comprend en outre des moyens de collecte et d'acheminement automatisés de la matière organique à méthaniser de l'élevage vers ladite zone de collecte et d'alimentation du dispositif de méthanisation.

Ces moyens de collecte et d'acheminement automatisés de la matière organique à méthaniser comprenant du fumier sont connus de l'homme du métier, et peuvent comprendre des systèmes, tels des racloirs couplés avec des systèmes automatiques à pistons, chargés de récupérer la matière organique comprenant du fumier pour l'acheminer par exemple sur un convoyeur jusqu'à ladite zone de collecte et d'alimentation.

Avantageusement, la matière organique est collectée directement dans le bâtiment d'élevage pour être acheminée vers la zone de collecte et d'alimentation de façon automatisée.

Les définitions et différentes variantes de réalisation décrites en référence à un premier aspect et/ou à un second aspect selon l'invention s'applique(nt) au troisième aspect selon l'invention.

### Description détaillée des dessins

La présente invention sera mieux comprise à la lecture des exemples de réalisation décrits ci-après et cités à titre non limitatifs, illustrés par les figures suivantes annexées à la présente, et dans lesquelles :
- La figure **1** est une représentation schématique et vue de dessus d'un premier exemple de dispositif de méthanisation selon l'invention ;
- La figure **2** est une représentation schématique selon le plan de coupe II-II représenté à la figure **1** du premier exemple de dispositif de méthanisation ;
- La figure **3** est une représentation schématique, vue de dessus, d'un second exemple de dispositif de méthanisation selon l'invention.
- Les figures **4** et **5** représentent une variante du dispositif de méthanisation représenté sur les figures 1 et 2.

### Description détaillée des exemples de réalisation

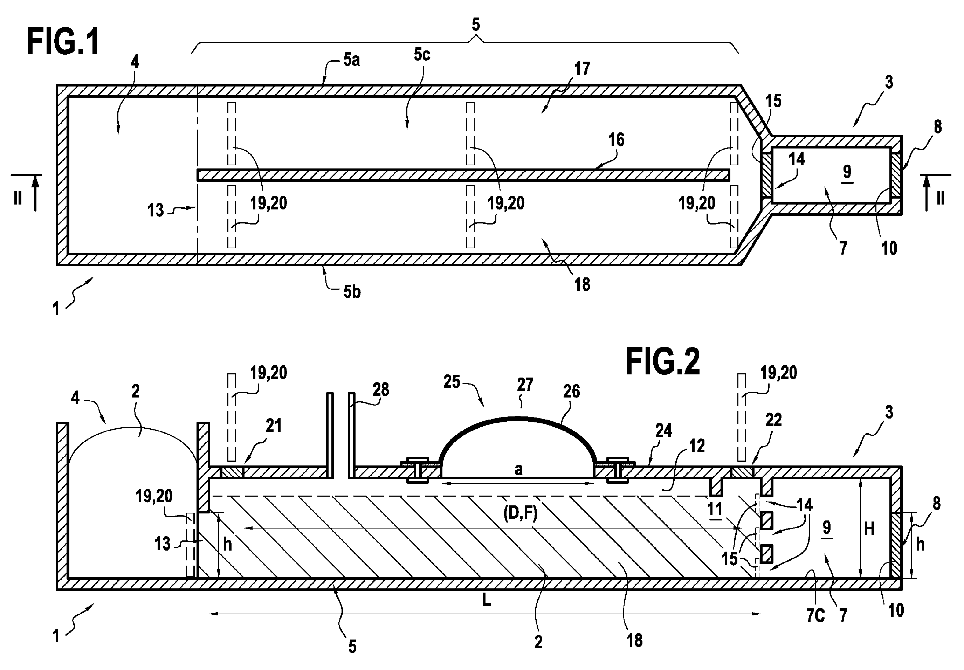

Le premier exemple de dispositif de méthanisation **1** par voie sèche de matière organique **2** selon l'invention, représenté aux figures **1** et **2****,** comprend un digesteur **3** comprenant une zone de collecte et d'alimentation **4** de la matière organique **2** à digérer, notamment apte à tenir en tas, un couloir de digestion **5** et une zone d'évacuation et de stockage tampon **7** de la matière organique digérée formant un digestat **9.** Ledit couloir de digestion **5** comprend une zone de génération de gaz **11** destinée à recevoir la matière organique **2** ayant été mouillée avec un fluide et une zone de collecte du gaz **12** disposée au-dessus de la zone de génération du gaz **11** dans ledit couloir de digestion **5** pour collecter le biogaz produit dans la zone de génération de gaz **11.** Ledit couloir de digestion **5** comprend également au moins un orifice d'admission **13** de la matière organique **2** à digérer pour son admission de la zone d'alimentation et de collecte **4** dans la zone de génération de gaz **11,** ledit orifice d'admission **13** comprenant éventuellement un dispositif de réglage de la taille de son ouverture amovible. Dans cet exemple précis, il n'y a pas de tel dispositif de réglage de la taille de l'ouverture de l'orifice d'admission **13.** Ledit couloir de digestion **5** comprend en outre trois orifices d'évacuation **14,** alignés verticalement, de la matière organique digérée **9** pour son évacuation de la zone de génération de gaz **11** dans la zone d'évacuation et de stockage tampon **7,** lesdits orifices d'évacuations **14** comprenant un dispositif de fermeture et d'ouverture amovible **15** permettant la fermeture ou l'ouverture simultanée desdits trois orifices **14** ou de manière différenciée. Cette disposition permet avantageusement une sortie de manière homogène de la matière organique digérée.

Selon un mode de réalisation, la zone d'évacuation et de stockage tampon **7** comprend également un orifice de sortie **8** de la matière organique digérée **9** (digestat) pourvue d'un dispositif de fermeture et d'ouverture amovible **10,** notamment identique au dispositif de fermeture et d'ouverture amovible **15** dudit au moins un orifice d'évacuation **14** du couloir de digestion **5**.

L'orifice de sortie **8** de la matière organique digérée **9** s'étend du fond **7c** de ladite zone d'évacuation et de stockage tampon **7.** Cette disposition facilite la sortie et donc l'évacuation de la matière organique digérée **9** en évitant que cette dernière ne sédimente en certains endroits de la zone d'évacuation et de stockage tampon **7.**

Le couloir de digestion **5** comprend une cloison de séparation **16** disposée dans ledit couloir de digestion **5** afin de former au moins deux chambres de digestion **17,18** en liaison fluidique l'une avec l'autre, laquelle cloison de séparation **16** comprend des moyens de chauffage pour le chauffage de la matière organique **2.**

Le couloir de digestion **5** est défini par des parois latérales gauche **5a** et droite **5b** et une paroi de fond **5c,** et comprend au moins un moyen de raclement **19** d'au moins l'une desdites parois latérales **5a,5b** ou de fond **5c** disposé librement. Dans cet exemple précis, le digesteur 3 comprend six moyens de raclement **19,** c'est-à-dire trois par chambre de digestion **(17,18).** Chaque moyen de raclement **19** est une grille **20** disposée librement dans la matière organique **2** et se déplace le long du couloir de digestion **5,** et donc dans la chambre de digestion **(17,18)** le recevant, sous l'effet du déplacement libre de la matière organique **2.** Dans une variante non représentée, le moyen de raclement **19** comprend deux grilles **20** reliées dans leurs parties supérieures par un bras de liaison, de sorte que dans la configuration représentée à la figure **2****,** les grilles **20** appairées se déplacent dans le couloir de digestion, une grille par chambre de digestion pour une paire de grilles. De préférence, dans cette variante, chaque grille comprend une partie supérieure et une partie inférieure montées sur des moyens de rappel en sorte que les grilles dans leur position repliée, correspondant à la position dans laquelle les parties supérieure et inférieure sont l'une contre l'autre, puissent être introduites dans la zone de collecte et d'alimentation **4** à l'entrée de l'orifice d'admission **13** tel que représenté en pointillés à la figure **2****.** Puis, sous l'effet du déplacement de la matière organique, les parties supérieure et inférieure de chaque grille s'écartent et se déploient dans un même plan correspondant à une position dépliée.

Le digesteur **3** comprend des moyens de chauffage disposés dans au moins l'une desdites parois latérales **5a,5b** et de fond **5c** et/ou la cloison de séparation **16,** ici dans chacune des parois latérales **5a,5b** et de fond **5c.** Le digesteur **3** comprend un module de cogénération (non représenté) comprenant un moteur de cogénération configuré pour être alimenté au moins en partie par le biogaz produit dans la zone de collecte **12** et pour alimenter en chaleur lesdits moyens de chauffage.

Le digesteur **3** comprend éventuellement une ouverture d'admission **21** des moyens de raclement **19,** disposée à proximité dudit orifice d'admission **13** du couloir de digestion **5,** et une ouverture de récupération **22** desdits moyens de raclement **19,** disposée près de l'orifice d'évacuation **14** du couloir de digestion **5**.

La partie supérieure du couloir de digestion **5** est fermée de manière étanche à l'aide d'un élément de toit **24** apte à fermer de manière amovible ledit couloir de digestion **5**. Lesdites ouvertures d'admission **21** et de récupération **22** des moyens de raclement **19** sont disposées dans ledit élément de toit **24.**

La hauteur de la cloison de séparation **16** est inférieure à la hauteur de l'élément de toit **24** en sorte de ménager un espace dans lequel le bras de liaison du moyen de raclement **19** reliant les grilles appairées **20** puisse être éventuellement reçu.

De préférence, la zone de collecte et d'alimentation **4,** le couloir de digestion **5,** et la zone de stockage tampon **7** sont en béton, notamment isolé thermiquement, et sont enterrés.

Tel que cela est visible à la figure **2****,** le digesteur **3** comprend selon une portion déterminée de la longueur L du couloir de digestion **5,** une zone d'évent **25** laquelle est recouverte par au moins une membrane imperméable au biogaz **26.**

A titre d'exemple, et de manière purement indicative et non limitative, la longueur L peut être d'environ 25 mètres, la hauteur H peut être d'environ 4-5 mètres et la hauteur h peut être d'environ 2 mètres. La taille d'un des orifices d'évacuation **14** peut être de l'ordre de 60 cm de diamètre.

En fonctionnement, la matière organique **2** comprenant du fumier est tout d'abord collectée et alimentée dans la zone de collecte et d'alimentation **4,** de préférence en sorte de former un tas, notamment ayant une forme de dôme. Puis, la matière organique **2** est arrosée à l'aide d'un fluide, notamment ayant une température supérieure ou égale à 35°C, en particulier jusqu'à écroulement du tas. La matière organique **2** ainsi mouillée est admise par l'orifice d'admission **13** dans le couloir de digestion **5,** en particulier dans la zone de génération **11,** et donc dans les deux chambres de digestion **(17,18)** disposées en parallèle. Les parois latérales gauche **5a** et droite **5c,** la paroi de fond **5c,** et la cloison de séparation **16** sont chauffées en sorte que la matière organique **2** soit également chauffée, de préférence à une température supérieure ou égale à 35°C, en particulier au moins de l'ordre de 37°C. Le chauffage du couloir de digestion **5** est alimenté en tout ou partie par le module de cogénération (non représenté) alimenté en tout ou partie par le biogaz produit par le dispositif de méthanisation **1.** La matière organique **2** mouillée est transférée sous l'effet de son propre poids entre les orifices d'admission **13** et d'évacuation **14** dans le couloir de digestion **5** selon l'axe (D), lequel est sensiblement parallèle à l'axe longitudinal (L) du couloir de digestion **5**. Le dispositif de fermeture et d'ouverture **15** ferme les orifices d'évacuation **14.** La matière organique **2,** mouillée avec ledit fluide, est digérée pendant au moins **20** jours dans le couloir de digestion **5,** le gaz produit est collecté dans la zone de collecte du gaz **11** par l'intermédiaire du conduit **28** représenté à la figure **2****,** lequel est indirectement relié au module de cogénération. Lorsque la matière organique **2** se déplace, elle entraîne les grilles **20** permettant le nettoyage des parois **5a,5b,5c** du couloir de digestion **5** ainsi que celles de la cloison de séparation **16** mais aussi l'homogénéisation de la matière organique **2** et son dégazage. L'opérateur peut facilement enlever les grilles **20** via l'ouverture de récupération **22** et/ou en ajouter via l'ouverture d'admission **21** de ces dernières ou encore par la zone de stockage et d'alimentation **4.**

Lorsque la matière organique est digérée, le digestat **9** est évacué par les orifices d'évacuation **14** vers la zone tampon de stockage **7,** après ouverture du dispositif de fermeture et d'ouverture **15,** lequel dispositif **15** comprend un organe de fermeture, par exemple une lame guillotine, apte à fermer les trois orifices d'évacuation, en se déplaçant selon une direction transversale, notamment perpendiculaire, à l'axe de déplacement (D) de la matière organique **2** mouillée, en particulier perpendiculairement à l'axe longitudinal (L) du couloir de digestion **5**.

Une fois la zone de stockage tampon **7** remplie du digestat **9,** le dispositif de fermeture et d'ouverture **10** est ouvert ce qui permet de récupérer le digestat **9.** La zone de stockage tampon **7** permet de contrôler le déplacement de la matière organique **2** et donc l'avancée de la matière organique **2** dans le couloir de digestion **5** et son degré de digestion en l'absence de moyens mécaniques d'avancée de la matière organique **2.** La zone d'évacuation et de stockage tampon **7** permet également de contrôler la poussée de la matière organique **2** mouillée sur les parois du couloir de digestion et de celles de la cloison de séparation **16** en ouvrant les orifices d'évacuation **14.**

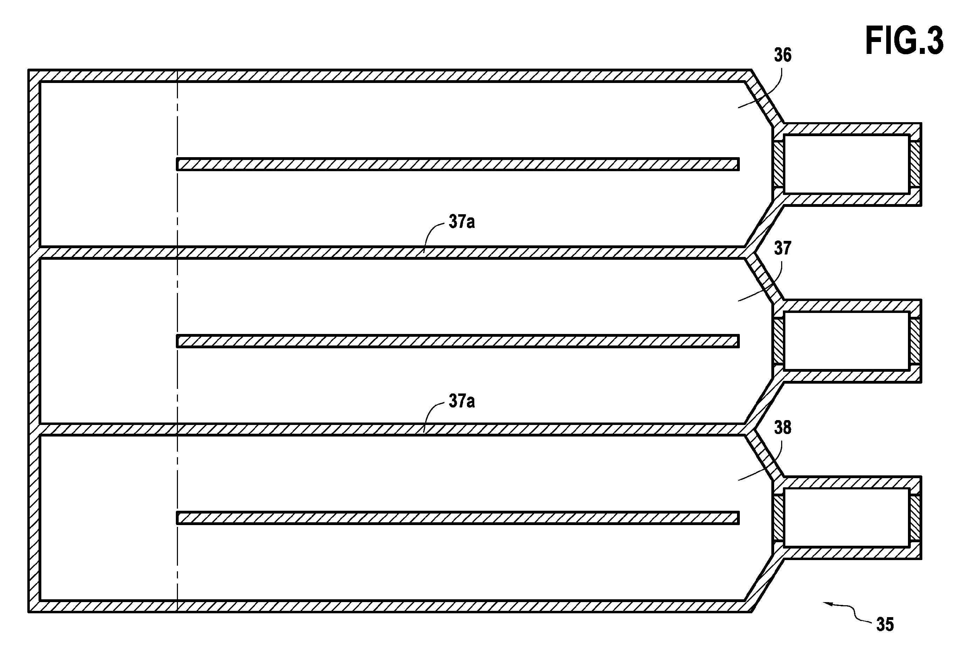

Le second exemple de dispositif de méthanisation **35** représenté à la figure **3** comprend trois digesteurs **36,37,38** selon l'invention, en particulier identique au digesteur **3** du premier exemple de dispositif **1** représenté et décrit ci-dessus.

Les trois digesteurs **36,37,38** sont montés en parallèle. Le digesteur central **37** comprend deux parois latérales communes **37a,37b** avec les digesteurs externes **36,38.** Ce dispositif de méthanisation **35** permet d'économiser de la chaleur, et donc de l'énergie, et de réguler l'alimentation en matière organique selon le nombre de digesteur en fonctionnement.

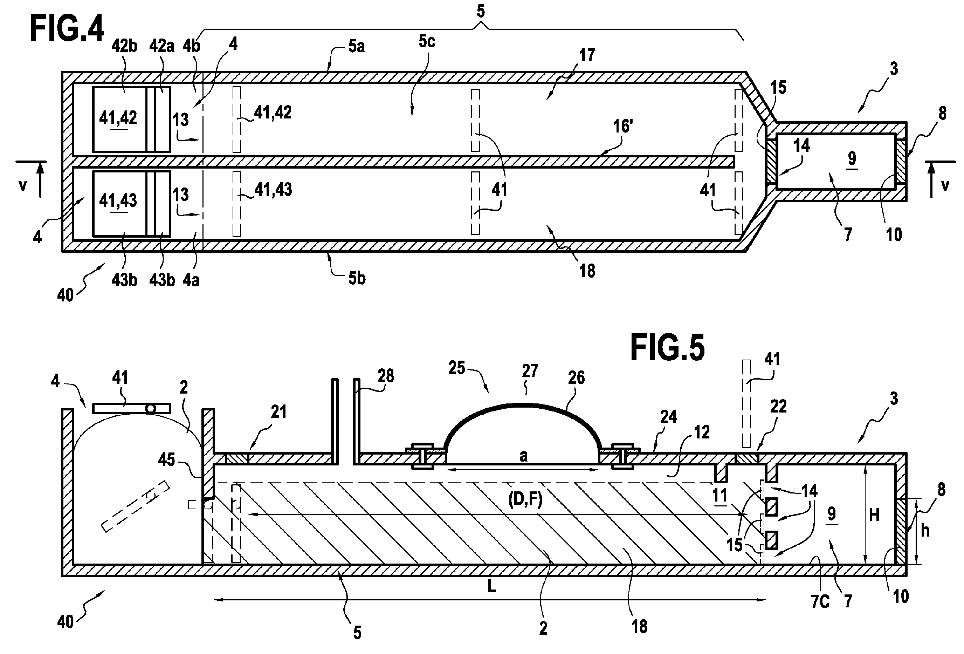

Les figures **4** et **5** représentent un dispositif de méthanisation **40,** qui est une variante du dispositif de méthanisation **1** représenté dans les figures **1** et **2****.** Ce dispositif **40** ne sera décrit ci-après que de par ses différences avec le dispositif de méthanisation **1.** Les références des figures **1** et **2** ont été reprises sur les figures **4** et **5** pour les caractéristiques ne changeant pas. Le dispositif de méthanisation **40** comprend une cloison de séparation **16'** s'étendant dans la zone de collecte et de stockage **4** en séparant cette dernière en deux sous-zones de collecte et de stockage **4a** et **4b.** Le dispositif **40** comprend un moyen de raclement **41** comprenant deux grilles **42** et **43,** indépendantes l'une de l'autre, et disposées chacune librement dans la matière organique à digérer dans la zone de collecte et de stockage **4.** En particulier, chaque sous-zone **4a** et **4b** reçoit au moins une grille **42,43.** Chaque grille **42,43** comprend une partie supérieure **42a, 43a** et une partie inférieure **42b, 43b** montées l'une par rapport à l'autre à l'aide d'un dispositif de rappel, tel un ressort de rappel, en sorte qu'en position déployée, et donc au repos, les parties supérieure et inférieure soient sensiblement dans un même plan, et qu'en position pliée, la partie supérieure soit apte à pivoter et se rabattre vers la partie inférieure.

Dans cette variante, l'ouverture d'admission **21** des moyens de raclement **41** dans le couloir de digestion **5** est optionnelle. Les moyens de raclement **40** sont en effet avantageusement introduits dans le couloir de digestion **5** en étant posés à plat sur la matière organique à digérer **2** dans la zone de collecte et de stockage **4.**

Le dispositif **40** comprend une cloison de déviation **45** disposée entre la zone de collecte et de stockage **4** et le couloir de digestion **5,** et délimitant ainsi lesdits orifices d'admission.

Lesdits orifices d'admission **13** ayant des dimensions inférieures à celles de la grille **42,43** mais supérieures à celles de sa partie supérieure **42a,43a** et/ou de sa partie inférieure **42b,43b.**

En fonctionnement, les grilles **42** et **43** sont disposées à plat dans la zone de collecte et de stockage **4,** en particulier une grille par sous-zone de stockage et de collecte, puis au fur et à mesure de l'avancée de la matière organique dans le couloir de digestion, les grilles **42,43** passent d'une position horizontale à une position verticale jusqu'à venir en butée contre la cloison de déviation **45.** Sous la force exercée par la matière organique à digérer **2** et venant en appui contre la cloison de déviation 45, la partie supérieure **42a,43a** de la grille est rabattue contre la partie inférieure **42b,43b.** Une fois, la grille passée dans le couloir de digestion, la partie supérieure revient dans sa position déployée, au repos, sous l'effet du dispositif de rappel, et est apte à racler au moins l'une des parois du couloir de digestion.

## Revendications

1. Dispositif (1,35,40) pour la méthanisation par voie sèche de matière organique (2), **caractérisé en ce qu'**il comprend au moins un digesteur (3) comprenant:
- au moins une zone de collecte et d'alimentation (4) de la matière organique (2) à digérer comprenant du fumier ;
- un couloir de digestion (5) comprenant :
- une zone de génération de gaz (11) destinée à recevoir la matière organique (2) ayant été mouillée avec un fluide ;
- une zone de collecte du gaz (12) disposée au-dessus de la zone de génération du gaz (11) dans ledit couloir de digestion (5) pour collecter le biogaz produit dans la zone de génération de gaz (11) ;
- une zone d'évacuation et de stockage tampon (7) de la matière organique digérée (9) ;
**en ce que** ledit couloir de digestion (5) comprend au moins un orifice d'admission (13) de la matière organique (2) à digérer pour son admission de la zone d'alimentation et de collecte (4) dans la zone de génération de gaz (11), ledit orifice d'admission (13) comprenant éventuellement un dispositif de réglage de la taille de son ouverture amovible ;
et **en ce que** ledit couloir de digestion (5) comprend au moins un orifice d'évacuation (14) de la matière organique digérée (9) pour son évacuation de la zone de génération de gaz (11) dans la zone d'évacuation et de stockage tampon (7), ledit orifice d'évacuation (14) comprenant un dispositif de fermeture et d'ouverture amovible (15).

2. Dispositif (1,35,40) selon la revendication 1, **caractérisé en ce que** la matière organique à digérer (2) comprend au moins 30% de fumier en masse par rapport à sa masse totale, notamment au moins 50% de fumier en masse par rapport à sa masse totale, ledit fumier comprenant de la paille et/ou de la menue paille, éventuellement mélangée(s) avec du lisier et/ou des déjections animales.

3. Dispositif (1,35) selon l'une ou l'autre des revendications 1 et 2, **caractérisé en ce que** le couloir de digestion (5) comprend des parois latérales gauche (5a) et droite (5b) et une paroi de fond (5c), et **en ce qu'**il comprend un moyen de raclement (19,20) d'au moins l'une desdites parois latérales (5a,5b) et de fond (5c) disposé librement.

4. Dispositif (1,35,40) selon la revendication 3, **caractérisé en ce que** ledit moyen de raclement (19,35) comprend au moins une grille (20,42,43) disposée librement dans la matière organique (2) et se déplaçant le long du couloir de digestion (5), de préférence dans au moins une chambre de digestion (17,18), sous l'effet du déplacement libre de la matière organique (2).

5. Dispositif (1,35,40) selon l'une quelconque des revendications 1 à 4, **caractérisé en ce que** la zone d'évacuation et de stockage tampon comprend également un orifice de sortie de la matière organique digérée, éventuellement pourvue d'un dispositif de fermeture et d'ouverture amovible.

6. Dispositif (1,35,40) selon l'une quelconque des revendications 1 à 5, **caractérisé en ce que** la zone d'évacuation et de stockage tampon est une fosse pourvue éventuellement d'un élément de toit amovible.

7. Dispositif (1,35) selon l'une quelconque des revendications 1 à 6, **caractérisé en ce que** le couloir de digestion (5) comprend au moins une cloison de séparation (16) disposée dans ledit couloir de digestion (5) afin de former au moins deux chambres de digestion (17,18) en liaison fluidique l'une avec l'autre, de préférence la cloison de séparation (16) comprend des moyens de chauffage pour le chauffage de la matière organique (2).

8. Dispositif (1,35) selon l'une quelconque des revendications 1 à 7, **caractérisé en ce qu'**il comprend des moyens de chauffage disposés dans au moins l'une desdites parois latérales (5a,5b) et de fond (5c) et/ou la cloison de séparation (16), et **en ce qu'**il comprend un moteur de cogénération configuré pour être alimenté au moins en partie par le biogaz produit dans la zone de collecte et pour alimenter en chaleur lesdits moyens de chauffage.

9. Dispositif (1,35) selon l'une quelconque des revendications 1 à 8, **caractérisé en ce qu'**il comprend une ouverture d'admission (21) dudit moyen de raclement (19), disposée à proximité dudit orifice d'admission (13) du couloir de digestion (5), et une ouverture de récupération (22) dudit moyen de raclement (19), disposée près dudit au moins un orifice d'évacuation (14) du couloir de digestion (5).

10. Dispositif (1,35) selon l'une quelconque des revendications 1 à 9, **caractérisé en ce que** le couloir de digestion (5) comprend au moins deux, de préférence au moins trois, orifices d'évacuation (14), alignés verticalement.

11. Dispositif (1,35) selon l'une quelconque des revendications 1 à 10, **caractérisé en ce que** la zone d'évacuation et de stockage tampon (7) comprend un orifice de sortie (8) de la matière organique (9) s'étendant du fond de ladite zone d'évacuation et de stockage tampon (7) pourvu d'un dispositif de fermeture et d'ouverture amovible (10).

12. Dispositif (35) selon l'une quelconque des revendications 1 à 11, **caractérisé en ce qu'**il comprend au moins deux digesteurs (36,37,38), lesdits deux digesteurs ayant au moins une paroi latérale commune (37a,37b).

13. Procédé de méthanisation par voie sèche de matière organique (2) **caractérisé en ce qu'**il comprend les étapes suivantes effectuées à l'aide du dispositif de méthanisation (1,35) selon l'une quelconque des revendications 1 à 12 :
a) une étape de collecte et d'alimentation de la matière organique (2) à digérer comprenant du fumier dans une zone de collecte et d'alimentation (4) ; et
b) une étape d'arrosage de la matière organique (2) disposée dans la zone de collecte et d'alimentation (4) à l'aide d'un fluide ; et
c) une étape de transfert de la matière organique (2) de la zone de collecte et d'alimentation (4) vers un couloir de digestion (5) ayant un orifice d'admission (13) de la matière organique (2) et au moins un orifice d'évacuation (14) de la matière organique (2,9), l'avancée de la matière organique (2) dans le couloir de digestion (5) entre les orifices d'admission (13) et d'évacuation (14) est obtenue par le déplacement gravitaire de la matière organique (2), le couloir de digestion (5) comprenant un dispositif de fermeture et d'ouverture amovible (15) dudit au moins un orifice d'évacuation (14) ; et
d) ledit couloir de digestion (5) comprend en outre une zone de génération de gaz (11) destinée à recevoir la matière organique (2) ayant été mouillée avec un fluide, et une zone de collecte du gaz (12) disposée au-dessus de la zone de génération du gaz (11) dans ledit couloir de digestion (5) pour collecter le biogaz produit dans la zone de génération de gaz (11) ; et
e) une étape de collecte du biogaz produit ; et
f) une étape de transfert de la matière organique digérée (9) formant un digestat du couloir de digestion (5) vers une zone d'évacuation et stockage tampon (7).

14. Installation **caractérisée en ce qu'**elle comprend un dispositif de méthanisation (1,35) selon l'une quelconque des revendications 1 à 12, et **en ce qu'**elle comprend des moyens de collecte et d'acheminement automatisés de la matière organique (2) à méthaniser de l'élevage vers ladite zone de collecte et d'alimentation (4) du dispositif de méthanisation (1,35).
